# EUROPEAN PATENT APPLICATION

(11) **EP 4 697 426 A1**
(43) Date of publication of application: **18.02.2026**
(21) Application number: 23933370.1
(22) Date of filing: 18.04.2023
(51) Int. Cl.: H01M 10/0567

(54) **ELECTROLYTE, SECONDARY BATTERY, AND ELECTRICAL APPARATUS**

(71) Applicant: Contemporary Amperex Technology Co., Limited, Ningde, Fujian 352100 (CN)
(72) Inventor: LIU, Jiao, Ningde, Fujian 352100 (CN); ZHANG, Limei, Ningde, Fujian 352100 (CN); CHEN, Peipei, Ningde, Fujian 352100 (CN); REN, Jiamo, Ningde, Fujian 352100 (CN); PENG, Chang, Ningde, Fujian 352100 (CN)
(74) Representative: Ziebig Hengelhaupt Intellectual Property Attorneys Patentanwaltskanzlei PartGmbB
(86) International application number: PCT/CN2023/088944
(87) International publication number: WO 2024/216490

(57) **Abstract**

This application provides an electrolyte, a secondary battery, and an electric apparatus. The electrolyte includes a cyclic sulfate compound represented by Formula I and a metal ion additive. The cyclic sulfate compound and the metal ion additive contribute to the formation of a stable SEI film, thereby facilitating an improvement in the rate performance of the battery.

## Description

### TECHNICAL FIELD

This application relates to the field of secondary battery technology, and in particular, to an electrolyte, a secondary battery, and an electric apparatus.

### BACKGROUND

In recent years, with the development of secondary battery technology, secondary batteries have been widely applied in energy storage power systems such as hydroelectric, thermal, wind, and solar power stations, as well as in various fields including power tools, electric bicycles, electric motorcycles, electric vehicles, military equipment, and aerospace. Due to the significant advancements in the application fields of secondary batteries, higher requirements have been imposed on the performance of the batteries.

During a charging process of a battery, an electrolyte continuously undergoes reduction at a negative electrode. To reduce side reactions at the negative electrode, attempts have been made to add various compounds to the electrolyte to form a passivation layer on the negative electrode surface, where the passivation layer is also known as a solid electrolyte interphase (SEI) film. Studies have shown that forming a uniform, dense, stable, low-impedance, and well-adhered solid electrolyte interphase (SEI) film with excellent properties is conducive to improving the electrochemical performance of the battery and improving the performance of the battery. However, existing electrolyte systems exhibit high impedance, failing to meet the demand for improved battery performance.

### SUMMARY

This application is made in view of the above issues, with an objective to provide an electrolyte. The electrolyte includes a cyclic sulfate compound represented by Formula I and a metal ion additive. The cyclic sulfate compound and the metal ion additive contribute to the formation of a stable SEI film, thereby facilitating an improvement in the rate performance of the battery.

According to a first aspect of this application, an electrolyte is provided, where the electrolyte includes a cyclic sulfate compound represented by Formula I and a metal ion additive; 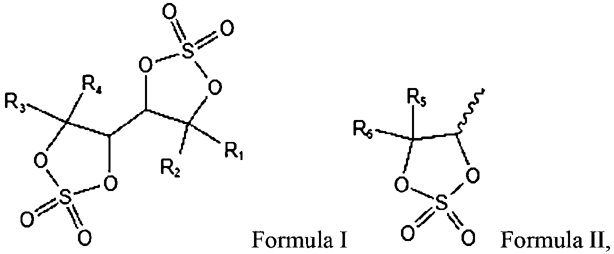
where in Formula I, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and R₁, R₂, R₃, and R₄ are not all selected from a hydrogen atom; and
in Formula II, R₅ and R₆ are each independently selected from a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and a wavy line indicates a bonding position.

On one hand, introducing the cyclic sulfate compound into the electrolyte facilitates the formation of a stable SEI film. During a charging process of a battery, the cyclic sulfate group contributes to the formation of an inorganic sulfite-based film between the electrolyte and a negative electrode plate; and the R₁ group, the R₂ group, the R₃ group, or the R₄ group contributes to the formation of an elastic organic film between the electrolyte and the negative electrode plate. For example, introducing an alkyl group/an alkoxy group into the R₁ group, the R₂ group, the R₃ group, or the R₄ group can generate an elastic SEI film with a longer organic chain on a negative electrode side, coping with volume changes on the negative electrode side during cycling to avoid or reduce damage to the SEI film, thereby improving the stability of the SEI film. Additionally, introducing an element including F or N into the R₁ group, the R₂ group, the R₃ group, or the R₄ group can participate in film formation on the negative electrode side, generating an SEI film rich in inorganic components such as LiF or Li₃N, enhancing the mechanical strength of the SEI film and further improving the stability of the SEI film on the negative electrode side. On the other hand, introducing the metal ion additive into the electrolyte facilitates synergistic interaction with the cyclic sulfate compound, contributing to the formation of an inorganic film, and further forming a stable SEI film, thereby further preventing electron tunneling, reducing interface impedance, and significantly improving the rate performance of the battery.

In any embodiment, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, and a cyano group.

Selecting R₁, R₂, R₃, and R₄ each independently from a structure represented by Formula II, a hydrogen atom, a C₁-C₆ alkyl group, a halogen atom, a C₁-C₃ haloalkyl group, a C₁-C₃ alkoxy group, and a cyano group facilitates the separation of R₁, R₂, R₃, or R₄ from the cyclic sulfate group during the charging process of the battery, forming an organic film, enhancing the interface stability between the electrolyte and the negative electrode plate, and improving the rate performance of the battery.

In any embodiment, R₁ is selected from a hydrogen atom and a C₁-C₃ alkyl group; R₂ is selected from a C₁-C₃ alkyl group, a halogen atom, a C₁-C₃ haloalkyl group, a cyano group, or a structure represented by Formula II; R₃ is selected from a hydrogen atom and a C₁-C₃ alkyl group; and R₄ is selected from a C₁-C₃ alkyl group, a halogen atom, a C₁-C₃ alkoxy group, or a structure represented by Formula II.

In any embodiment, R₁ is selected from a hydrogen atom or a methyl group; R₂ is selected from a methyl group, an ethyl group, a fluorine atom, a trifluoromethyl group, a cyano group, or a structure represented by Formula II; R₃ is selected from a hydrogen atom; and R₄ is selected from a methyl group, an ethyl group, a propyl group, a fluorine atom, an ethoxy group, or a structure represented by Formula II.

In any embodiment, R₁ and R₃ are each a hydrogen atom; R₂ and R₄ are each a methyl group, an ethyl group, a fluorine atom, or a structure represented by Formula II; or R₂ is a methyl group or a structure represented by Formula II, and R₄ is a methyl group.

In any embodiment, R₁ and R₃ are each a hydrogen atom; and R₂ and R₄ are each a methyl group or a structure represented by Formula II.

In any embodiment, R₅ and R₆ are each independently selected from a hydrogen atom and a C₁-C₃ alkyl group.

In any embodiment, the cyclic sulfate compound is selected from one or more of the following compounds:

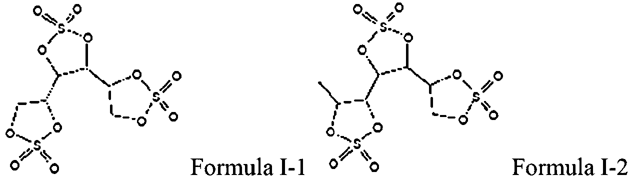

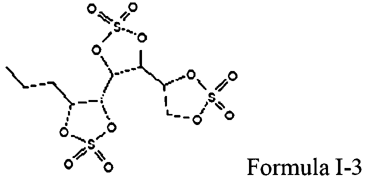

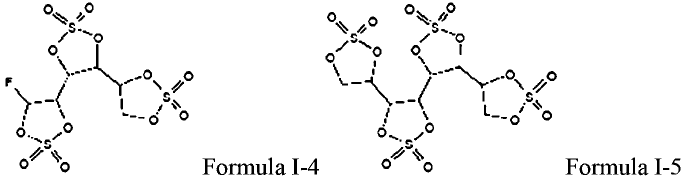

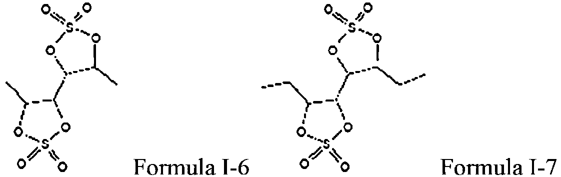

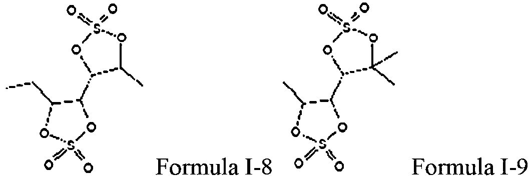

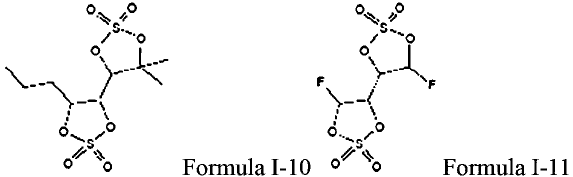

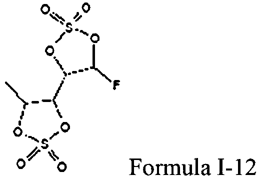

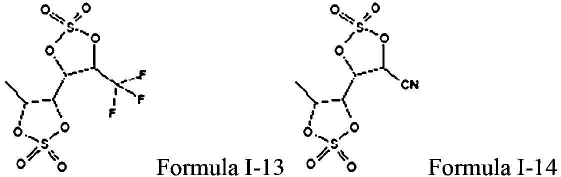

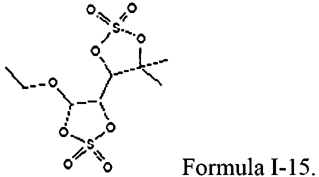

Adding the above cyclic sulfate compounds to the electrolyte contributes to the formation of an SEI film with a mixture of organic and inorganic films, forming a more stable interface, and improving the rate performance of the battery.

In any embodiment, based on a total mass of the electrolyte, a mass content W of the cyclic sulfate compound satisfies: 0.001% ≤ W ≤ 20%, optionally 0.1% to 5%.

Controlling the mass content of the cyclic sulfate compound within an appropriate range can not only meet the demand for improving the rate performance of the battery but also avoid or reduce the impact of an excessively high mass content of the cyclic sulfate compound on the ion transport rate.

In any embodiment, the metal ion additive includes one or more of alkali metal ions, alkaline earth metal ions, and high-valence metal ions.

In any embodiment, the alkali metal ions include one or more of sodium ions, lithium ions, and potassium ions; and/or
based on the total mass of the electrolyte, a mass content of the alkali metal ions is 30 ppm to 3000 ppm, optionally 50 ppm to 2000 ppm.

Introducing the alkali metal ions into the electrolyte contributes to the formation of an inorganic SEI film with stronger electron-blocking capability, and controlling the mass content of the alkali metal ions enables the formation of a stable SEI film, avoiding or reducing the impact of the introduction of the alkali metal ions on an electrolyte system.

In any embodiment, the high-valence metal ions include one or more of aluminum ions and copper ions; and/or
based on the total mass of the electrolyte, a mass content of the high-valence metal ions is 0.5 ppm to 300 ppm, optionally 15 ppm to 200 ppm.

Introducing the high-valence metal ions into the electrolyte contributes to the formation of an inorganic SEI film with stronger electron-blocking capability, and controlling the mass content of the high-valence metal ions enables the formation of a stable SEI film, avoiding or reducing the impact of the introduction of the high-valence metal ions on the electrolyte system.

In any embodiment, the alkaline earth metal ions include one or more of magnesium ions and calcium ions; and/or
based on the total mass of the electrolyte, a mass content of the alkaline earth metal ions is 0.5 ppm to 500 ppm, optionally 13 ppm to 400 ppm.

Introducing the alkaline earth metal ions into the electrolyte contributes to the formation of an inorganic SEI film with stronger electron-blocking capability, and controlling the mass content of the alkaline earth metal ions enables the formation of a stable SEI film, avoiding or reducing the impact of the introduction of the alkaline earth metal ions on the electrolyte system.

In any embodiment, the electrolyte further includes an electrolytic salt, where the electrolytic salt includes a lithium salt or a sodium salt;
the lithium salt includes one or more of lithium hexafluorophosphate, lithium perchlorate, lithium tetrafluoroborate, lithium hexafluoroarsenate, lithium bis(fluorosulfonyl)imide, lithium bis(trifluoromethanesulfonyl)imide, lithium difluorobis(oxalato)phosphate, and lithium bis(oxalate)borate; and
the sodium salt includes one or more of sodium hexafluorophosphate, sodium difluoro(oxalate)borate, sodium perchlorate, sodium bis(fluorosulfonyl)imide, sodium bis(trifluoromethanesulfonyl)imide, and sodium trifluoromethanesulfonate.

The above lithium salts or sodium salts have excellent compatibility with the cyclic sulfate compound, and introducing an appropriate amount of the cyclic sulfate compound into the electrolyte does not affect the lithium salts or sodium salts.

According to a second aspect of this application, a secondary battery is provided, including a negative electrode plate and the electrolyte described in the first aspect.

In any embodiment, the secondary battery includes a sodium battery or a lithium battery.

In any embodiment, the negative electrode plate includes a negative electrode active material, where the negative electrode active material includes one or more of artificial graphite, natural graphite, hard carbon, soft carbon, mesocarbon microbeads, carbon fiber, carbon nanotubes, elemental silicon, a silicon-oxygen compound, a silicon-carbon composite, a silicon alloy, elemental tin, a tin-oxygen compound, and a titanium composite material.

The above negative electrode active materials each have an excellent specific capacity and a high specific surface area, enabling the battery to have a high energy density.

In any embodiments, an active specific surface area A of the negative electrode plate and the mass content W of the cyclic sulfate compound satisfy: 0.0002 cm²/g ≤ W × A ≤ 0.04 cm²/g, optionally 0.02 cm²/g ≤ W × A ≤ 0.1 cm²/g.

When the active specific surface area A of the negative electrode plate and the mass content W of the cyclic sulfate compound satisfy a specified relationship, a stable SEI film can be formed, and the rate performance and cycling performance of the battery are balanced, fully utilizing the battery capacity.

In any embodiment, the active specific surface area A of the negative electrode plate satisfies: A ≤ 20 cm²/g, optionally 5 cm²/g to 15 cm²/g.

Controlling the active specific surface area A of the negative electrode plate within an appropriate range can avoid or reduce increased battery polarization due to an excessively small active specific surface area of the negative electrode plate so as not to affect the utilization of the battery capacity, and can also avoid or reduce increased interface side reactions due to an excessively large active specific surface area of the negative electrode plate so as not to affect the cycling performance and storage performance of the battery. An active specific surface area A within an appropriate range can balance the cycling performance, storage performance, and capacity of the battery.

According to a third aspect of this application, an electric apparatus is provided, including the secondary battery described in the second aspect.

### BRIEF DESCRIPTION OF DRAWINGS

FIG. 1 is a schematic diagram of a secondary battery cell according to an embodiment of this application;
FIG. 2 is an exploded view of the secondary battery cell according to the embodiment of this application shown in FIG. 1;
FIG. 3 is a schematic diagram of a battery module according to an embodiment of this application;
FIG. 4 is a schematic diagram of a battery pack according to an embodiment of this application;
FIG. 5 is an exploded view of the battery pack according to the embodiment of this application shown in FIG. 4; and
FIG. 6 is a schematic diagram of an electric apparatus using a secondary battery as a power source according to an embodiment of this application.

### Description of reference signs:

1. battery pack; 2. upper box body; 3. lower box body; 4. battery module; 5. secondary battery cell; 51. housing; 52. electrode assembly; and 53. cover plate.

### DESCRIPTION OF EMBODIMENTS

Embodiments that specifically disclose an electrolyte, a secondary battery, and an electric apparatus in this application are described in detail below with reference to the accompanying drawings as appropriate. However, unnecessary detailed descriptions may be omitted. For example, detailed descriptions of well-known matters or repetitive descriptions of substantially identical structures may be omitted. This is to avoid unnecessarily prolonging the following descriptions, for ease of understanding by persons skilled in the art. Additionally, the accompanying drawings and the following descriptions are provided for persons skilled in the art to fully understand this application and are not intended to limit the subject described in the claims.

"Ranges" disclosed in this application are defined in the form of lower and upper limits. A given range is defined by one lower limit and one upper limit selected, where the selected lower and upper limits define boundaries of that particular range. Ranges defined in this way may or may not include end values, and any combination may be used, meaning that any lower limit may be combined with any upper limit to form a range. For example, if ranges of 60-120 and 80-110 are provided for a specific parameter, it is understood that ranges of 60-110 and 80-120 can also be envisioned. Additionally, if minimum values of a range are given as 1 and 2, and maximum values of the range are given as 3, 4, and 5, the following ranges can all be envisioned: 1-3, 1-4, 1-5, 2-3, 2-4, and 2-5. In this application, unless otherwise specified, a value range of "a-b" is a short representation of any combination of real numbers between a and b, where both a and b are real numbers. For example, a value range of "0-5" means that all real numbers in the range of "0-5" are listed herein, and "0-5" is just an abbreviated representation of a combination of these numbers. Additionally, a parameter expressed as an integer greater than or equal to 2 is equivalent to disclosure that the parameter is, for example, an integer among 2, 3, 4, 5, 6, 7, 8, 9, 10, 11, 12, and so on.

Unless otherwise specified, all embodiments and optional embodiments of this application can be combined with each other to form new technical solutions.

Unless otherwise stated, all the technical features and optional technical features of this application can be combined with each other to form new technical solutions.

Unless otherwise specified, all steps of this application can be performed sequentially or randomly, preferably sequentially. For example, a method including steps (a) and (b) indicates that the method may include steps (a) and (b) performed sequentially or steps (b) and (a) performed sequentially. For example, the foregoing method may further include step (c), indicating that step (c) may be added to the method in any ordinal position, for example, the method may include steps (a), (b), and (c), steps (a), (c), and (b), steps (c), (a), and (b), or the like.

Unless otherwise specified, "include" and "contain" mentioned in this application are inclusive or may be exclusive. For example, "include" and "contain" may indicate that other components not listed may also be included or contained, or only the listed components may be included or contained.

Unless otherwise specified, in this application, the term "or" is inclusive. For example, the phrase "A or B" means "A, B, or both A and B". More specifically, any one of the following conditions satisfies the condition "A or B": A is true (or present) and B is false (or not present); A is false (or not present) and B is true (or present); or both A and B are true (or present).

Currently, an electrolyte faces numerous issues. Taking a lithium battery as an example, an electrolyte typically includes an ester solvent (for example, vinylene carbonate). The ester solvent is prone to side reactions with lithium at an interface, forming an SEI film rich in organic components such as alkyl lithium, leading to increased impedance and affecting the power performance of the battery. In addition, high impedance may lead to lithium precipitation at a negative electrode, further deteriorating the lifespan of a battery cell. Therefore, there is a need to design an electrolyte to meet the application requirements of next-generation electrochemical systems.

### [Electrolyte]

Based on this, this application provides an electrolyte, where the electrolyte includes a cyclic sulfate compound represented by Formula I and a metal ion additive; 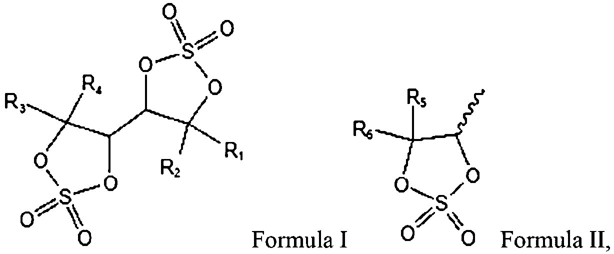
where in Formula I, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and R₁, R₂, R₃, and R₄ are not all selected from a hydrogen atom; and
in Formula II, R₅ and R₆ are each independently selected from a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and a wavy line indicates a bonding position.

In this specification, the term "C₁-C₆ alkyl group" refers to a linear or branched hydrocarbon chain group composed solely of carbon and hydrogen atoms, where the group has no unsaturation, has one to six carbon atoms, and is attached to the rest of the molecule via a single bond. Examples of the C₁-C₆ alkyl group include but are not limited to a methyl group, an ethyl group, an n-propyl group, a 1-methylethyl (isopropyl) group, an n-butyl group, an n-pentyl group, and an n-hexyl group.

In this specification, the term "cyclic sulfate compound" refers to a cyclic compound containing a sulfate group, where two oxygen atoms and a sulfur atom in the sulfate group serve as ring atoms in a five-membered ring structure.

In this specification, the term "metal ion additive" refers to a salt added to the electrolyte with a content of less than or equal to 5%, composed entirely of metal cations and anions, and presenting a liquid state at room temperature or a temperature near room temperature; metal cations include but are not limited to sodium ions, lithium ions, potassium ions, aluminum ions, copper ions, magnesium ions, or calcium ions. Room temperature refers to 25°C ± 5°C.

In this specification, the term "C₁-C₃ alkyl group" refers to a linear or branched hydrocarbon chain group composed solely of carbon and hydrogen atoms, where the group has no unsaturation, has one to three carbon atoms, and is attached to the rest of the molecule via a single bond. Examples of the C₁-C₃ alkyl group include but are not limited to a methyl group, an ethyl group, an n-propyl group, and a 1-methylethyl (isopropyl) group.

In this specification, the term "halogen atom" refers to elements in Group VIIA of the periodic table, including but not limited to F, Cl, Br, I, and At.

In this specification, the term "C₁-C₃ haloalkyl group" refers to a C₁-C₃ alkyl group in which at least one hydrogen atom is substituted with a halogen atom, including but not limited to trichloromethane, difluoroethylene, and 3-fluoropropene.

In this specification, the term "C₁-C₃ alkoxy group" refers to a group composed of a C₁-C₃ alkyl group and one oxygen atom, including but not limited to a methoxy group (CH₃O-), an ethoxy group (C₂H₅O-), and a propoxy group (C₃H₇O-).

In this specification, the term "C₁-C₃ haloalkoxy group" refers to a C₁-C₃ alkoxy group in which at least one hydrogen atom is substituted with a halogen atom, including but not limited to 1,1,1,2,2-pentafluoro-2-(2,2,2-trifluoroethoxy)ethane, 1-(2,2-difluoroethoxy)-2-(2,2,2-trifluoroethoxy)ethane, and 1,2-bis(2-fluoroethoxy)ethane.

In this specification, the term "double bond" refers to a double bond formed by two shared electron pairs between two atoms in a compound molecule, that is, a carbon-carbon double bond.

In this specification, the term "ester group" refers to a -COOR₁₀ group, where R₁₀ is selected from a substituted or unsubstituted C₁₋₅ alkyl group. In this specification, the term "C₁-C₅ alkyl group" refers to a linear or branched hydrocarbon chain group composed solely of carbon and hydrogen atoms, where the group has no unsaturation, has one to five carbon atoms, and is attached to the rest of the molecule via a single bond.

In this specification, the term "cyano group" refers to a -CN group.

In this specification, the term "sulfonic acid group" refers to a -SO₃H group.

In this specification, the term "bonding position" refers to a position where the structure represented by Formula II bonds with the structure represented by Formula I.

On one hand, introducing the cyclic sulfate compound into the electrolyte facilitates the formation of a stable SEI film. During a charging process of a battery, the cyclic sulfate group contributes to the formation of an inorganic sulfite-based film between the electrolyte and a negative electrode plate; the R₁ group, the R₂ group, the R₃ group, or the R₄ group contributes to the formation of an elastic organic film between the electrolyte and the negative electrode plate. For example, introducing an alkyl group or an alkoxy group into the R₁ group, the R₂ group, the R₃ group, or the R₄ group can generate an elastic SEI film with a longer organic chain on a negative electrode side, coping with volume changes on the negative electrode side during cycling to avoid or reduce damage to the SEI film, thereby improving the stability of the SEI film. Additionally, introducing an element including F or N into the R₁ group, the R₂ group, the R₃ group, or the R₄ group can participate in film formation on the negative electrode side, generating an SEI film rich in inorganic components such as LiF or Li₃N, enhancing the mechanical strength of the SEI film and further improving the stability of the SEI film on the negative electrode side. On the other hand, introducing the metal ion additive into the electrolyte facilitates synergistic interaction with the cyclic sulfate compound, contributing to the formation of an inorganic film, and further forming a stable SEI film, thereby further preventing electron tunneling, reducing interface impedance, and significantly improving the rate performance of the battery.

On the other hand, introducing the metal ion additive into the electrolyte facilitates synergistic interaction with the cyclic sulfate compound, contributing to the formation of an inorganic film, and further forming a stable SEI film, thereby further preventing electron tunneling, reducing interface impedance, and significantly improving the rate performance of the battery.

In some embodiments, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, and a cyano group.

Selecting R₁, R₂, R₃, and R₄ each independently from a structure represented by Formula II, a hydrogen atom, a C₁-C₆ alkyl group, a halogen atom, a C₁-C₃ haloalkyl group, a C₁-C₃ alkoxy group, and a cyano group facilitates the separation of R₁, R₂, R₃, or R₄ from the cyclic sulfate group during the charging process of the battery, forming an organic film, enhancing the interface stability between the electrolyte and the negative electrode plate, and improving the rate performance of the battery.

In some embodiments, R₁ is selected from a hydrogen atom and a C₁-C₃ alkyl group; R₂ is selected from a C₁-C₃ alkyl group, a halogen atom, a C₁-C₃ haloalkyl group, a cyano group, or a structure represented by Formula II; R₃ is selected from a hydrogen atom and a C₁-C₃ alkyl group; and R₄ is selected from a C₁-C₃ alkyl group, a halogen atom, a C₁-C₃ alkoxy group, or a structure represented by Formula II.

In some embodiments, R₁ is selected from a hydrogen atom or a methyl group; R₂ is selected from a methyl group, an ethyl group, a fluorine atom, a trifluoromethyl group, a cyano group, or a structure represented by Formula II; R₃ is selected from a hydrogen atom; and R₄ is selected from a methyl group, an ethyl group, a propyl group, a fluorine atom, an ethoxy group, or a structure represented by Formula II.

In some embodiments, R₁ and R₃ are each a hydrogen atom; R₂ and R₄ are each a methyl group, an ethyl group, a fluorine atom, or a structure represented by Formula II; or R₂ is a methyl group or a structure represented by Formula II, and R₄ is a methyl group.

In some embodiments, R₁ and R₃ are each a hydrogen atom; and R₂ and R₄ are each a methyl group or a structure represented by Formula II.

In some embodiments, R₅ and R₆ are each independently selected from a hydrogen atom and a C₁-C₃ alkyl group.

In some embodiments, the cyclic sulfate compound is selected from one or more of the following compounds:

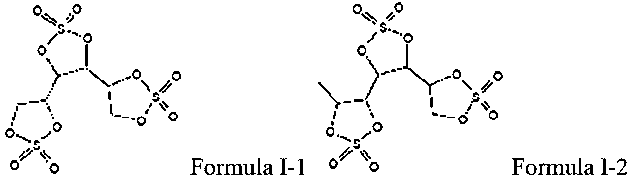

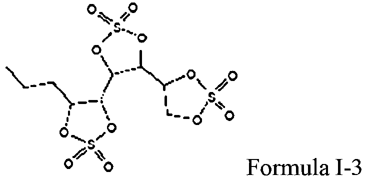

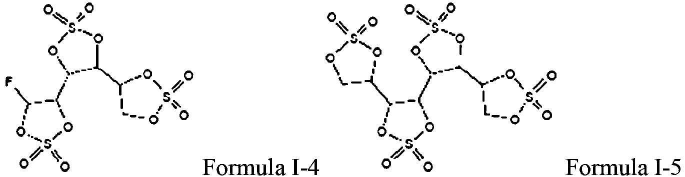

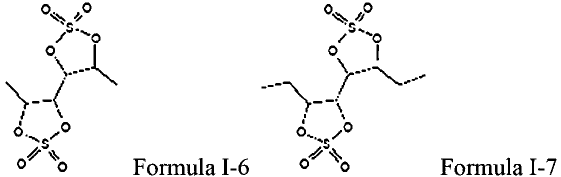

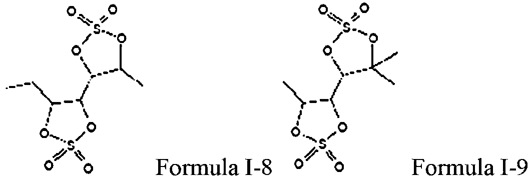

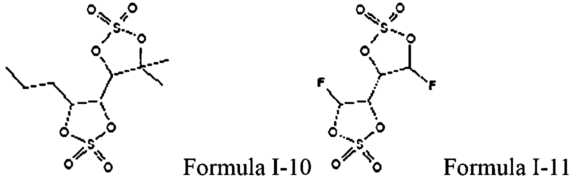

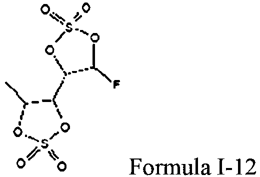

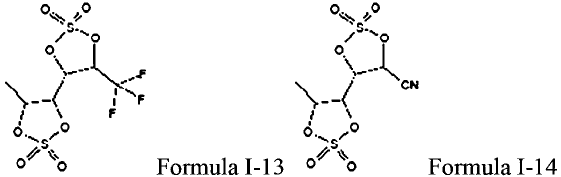

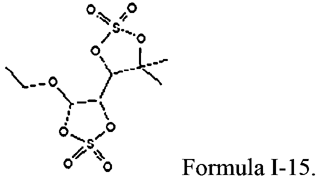

Adding the above cyclic sulfate compounds to the electrolyte contributes to the formation of an SEI film with a mixture of organic and inorganic films, forming a more stable interface, and improving the rate performance of the battery.

In some embodiments, based on a total mass of the electrolyte, a mass content W of the cyclic sulfate compound satisfies: 0.001% ≤ W ≤ 20%, optionally 0.1% to 5%.

In some embodiments, based on the total mass of the electrolyte, the mass content W of the cyclic sulfate compound is optionally 0.001%, 0.005%, 0.01%, 0.05%, 0.1%, 0.2%, 0.4%, 0.5%, 0.6%, 0.8%, 1%, 2%, 4%, 5%, 6%, 8%, 10%, 12%, 14%, 15%, 16%, 18%, 20%, or a value within a range defined by any two of the above values.

Controlling the mass content of the cyclic sulfate compound within an appropriate range can not only meet the demand for improving the rate performance of the battery but also reduce or decrease the impact of an excessively high mass content of the cyclic sulfate compound on the ion transport rate.

In some embodiments, the metal ion additive includes one or more of alkali metal ions, alkaline earth metal ions, and high-valence metal ions.

In some embodiments, the alkali metal ions include one or more of sodium ions, lithium ions, and potassium ions.

In some embodiments, based on the total mass of the electrolyte, a mass content of the alkali metal ions is 30 ppm to 3000 ppm, optionally 50 ppm to 2000 ppm.

In some embodiments, based on the total mass of the electrolyte, the mass content of the alkali metal ions is optionally 30 ppm, 50 ppm, 100 ppm, 200 ppm, 400 ppm, 500 ppm, 600 ppm, 800 ppm, 1000 ppm, 1500 ppm, 2000 ppm, 2500 ppm, 3000 ppm, or a value within a range defined by any two of the above values.

It can be understood that the alkali metal ions in the metal ion additive can participate in the formation of the SEI film such that some of the alkali metal ions are consumed, reducing the mass content of the alkali metal ions in the electrolyte. For example, the mass content of the alkali metal ions in the electrolyte is 30 ppm. Thus, based on the total mass of the electrolyte, the mass content of the alkali metal ions being 30 ppm to 3000 ppm is within the scope protected by the embodiments of this application.

Introducing the alkali metal ions into the electrolyte contributes to the formation of an inorganic SEI film with stronger electron-blocking capability, and controlling the mass content of the alkali metal ions enables the formation of a stable SEI film, avoiding or reducing the impact of the introduction of the alkali metal ions on an electrolyte system.

In some embodiments, the high-valence metal ions include one or more of aluminum ions and copper ions.

In some embodiments, based on the total mass of the electrolyte, a mass content of the high-valence metal ions is 0.5 ppm to 300 ppm, optionally 15 ppm to 200 ppm.

In some embodiments, based on the total mass of the electrolyte, the mass content of the high-valence metal ions is optionally 0.5 ppm, 1 ppm, 5 ppm, 10 ppm, 15 ppm, 20 ppm, 30 ppm, 40 ppm, 50 ppm, 60 ppm, 70 ppm, 80 ppm, 90 ppm, 100 ppm, 150 ppm, 200 ppm, 250 ppm, 300 ppm, or a value within a range defined by any two of the above values.

It can be understood that the high-valence metal ions in the metal ion additive can participate in the formation of the SEI film such that some of the high-valence metal ions are consumed, reducing the mass content of the high-valence metal ions in the electrolyte. For example, the mass content of the high-valence metal ions in the electrolyte is 0.5 ppm. Thus, based on the total mass of the electrolyte, the mass content of the high-valence metal ions being 0.5 ppm to 300 ppm is within the scope protected by the embodiments of this application.

Introducing the high-valence metal ions into the electrolyte contributes to the formation of an inorganic SEI film with stronger electron-blocking capability, and controlling the mass content of the high-valence metal ions enables the formation of a stable SEI film, reducing or decreasing the impact of the introduction of the high-valence metal ions on the electrolyte system.

In some embodiments, the alkaline earth metal ions include one or more of magnesium ions and calcium ions.

In some embodiments, based on the total mass of the electrolyte, a mass content of the alkaline earth metal ions is 0.5 ppm to 500 ppm, optionally 13 ppm to 400 ppm.

In some embodiments, based on the total mass of the electrolyte, the mass content of the alkaline earth metal ions is optionally 0.5 ppm, 1 ppm, 5 ppm, 10 ppm, 13 ppm, 15 ppm, 20 ppm, 30 ppm, 40 ppm, 50 ppm, 60 ppm, 70 ppm, 80 ppm, 90 ppm, 100 ppm, 150 ppm, 200 ppm, 250 ppm, 300 ppm, 350 ppm, 400 ppm, 450 ppm, 500 ppm, or a value within a range defined by any two of the above values.

It can be understood that the alkaline earth metal ions in the metal ion additive can participate in the formation of the SEI film such that some of the alkaline earth metal ions are consumed, reducing the mass content of the alkaline earth metal ions in the electrolyte. For example, the mass content of the alkaline earth metal ions in the electrolyte is 0.5 ppm. Thus, based on the total mass of the electrolyte, the mass content of the alkaline earth metal ions being 0.5 ppm to 500 ppm is within the scope protected by the embodiments of this application.

Introducing the alkaline earth metal ions into the electrolyte contributes to the formation of an inorganic SEI film with stronger electron-blocking capability, and controlling the mass content of the alkaline earth metal ions enables the formation of a stable SEI film, reducing or decreasing the impact of the introduction of the alkaline earth metal ions on the electrolyte system.

Metal ions may be introduced into the electrolyte by adding salts containing the selected metal ions, and may be hexafluorophosphate, for example, sodium hexafluorophosphate.

A mass content of metal ions in the metal ion additive in the electrolyte can be tested using instruments and methods well-known in the art, where the instruments include but are not limited to an ICP (Inductive Coupled Plasma Emission Spectrometer) inductive coupled plasma emission spectrometer; and testing is performed in accordance with the standard operating procedures recommended by the instrument manufacturer or industry standards in the art.

In some embodiments, the electrolyte further includes an electrolytic salt, where the electrolytic salt includes a lithium salt or a sodium salt;
the lithium salt includes one or more of lithium hexafluorophosphate (LiPF₆), lithium perchlorate (LiClO₄), lithium tetrafluoroborate (LiBF₄), lithium hexafluoroarsenate (LiAsF₆), lithium bis(fluorosulfonyl)imide (LiFSI), lithium bis(trifluoromethanesulfonyl)imide (LiTFSI), lithium difluorobis(oxalato)phosphate (LiDFOP), and lithium bis(oxalate)borate (LiBOB); and
the sodium salt includes one or more of sodium hexafluorophosphate (NaPF₆), sodium difluoro(oxalate)borate (NaDFOB), sodium perchlorate (NaClO₄), sodium bis(fluorosulfonyl)imide (NaFSI), sodium bis(trifluoromethanesulfonyl)imide (NaTFSI), and sodium trifluoromethanesulfonate (NaOTf).

In some embodiments, a concentration of the electrolytic salt is 0.1 mol/L to 5 mol/L, optionally 0.7 mol/L to 1.2 mol/L. In some embodiments, the concentration of the electrolytic salt is optionally 0.1 mol/L, 0.2 mol/L, 0.4 mol/L, 0.5 mol/L, 0.7 mol/L, 0.8 mol/L, 1 mol/L, 1.5 mol/L, 2 mol/L, 2.5 mol/L, 3 mol/L, 3.5 mol/L, 4 mol/L, 5.5 mol/L, 5 mol/L, or a value within a range defined by any two of the above values.

The above lithium salts or sodium salts have excellent compatibility with the cyclic sulfate compound, and introducing an appropriate amount of the cyclic sulfate compound into the electrolyte does not affect the lithium salts or sodium salts.

In some embodiments, the electrolyte further optionally includes an additive. For example, the additive may include a negative electrode film-forming additive and a positive electrode film-forming additive, and may further include an additive capable of improving some performance of a battery, for example, an additive for improving overcharge performance of the battery, or an additive for improving high-temperature performance or low-temperature performance of the battery.

### [Positive electrode plate]

The positive electrode plate includes a positive electrode current collector and a positive electrode film layer disposed on at least one surface of the positive electrode current collector, where the positive electrode film layer includes a positive electrode active material, a conductive agent, and a binder.

As an example, the positive electrode current collector has two opposite surfaces in its thickness direction, and the positive electrode film layer is disposed on either or both of the two opposite surfaces of the positive electrode current collector.

In some embodiments, the positive electrode current collector may be a metal foil or a composite current collector. For example, an aluminum foil may be used as the metal foil. The composite current collector may include a polymer material substrate and a metal layer formed on at least one surface of the polymer material substrate. The composite current collector may be formed by forming a metal material (for example, aluminum, aluminum alloy, nickel, nickel alloy, titanium, titanium alloy, silver, or silver alloy) on a polymer material substrate (for example, polypropylene (PP), polyethylene terephthalate (PET), polybutylene terephthalate (PBT), polystyrene (PS), or polyethylene (PE)).

In some embodiments, the positive electrode active material may be a well-known positive electrode active material used for batteries in the art. As an example, the positive electrode active material may include at least one of the following materials: olivine-structured lithium-containing phosphate, lithium metal oxides, and their respective modified compounds. However, this application is not limited to these materials, and other conventional materials that can be used as positive electrode active materials for batteries may also be used. These positive electrode active materials may be used alone or in combination of two or more. Examples of lithium metal oxides may include but are not limited to at least one of lithium cobalt oxide (for example, LiCoO₂), lithium nickel oxide (for example, LiNiO₂), lithium manganese oxide (for example LiMnO₂ or LiMn₂O₄), lithium nickel cobalt oxide, lithium manganese cobalt oxide, lithium nickel manganese oxide, lithium nickel cobalt manganese oxide (for example, LiNi_{1/3}Co_{1/3}Mn_{1/3}O₂ (also referred to as NCM333), LiNi_{0.5}Co_{0.2}Mn_{0.3}O₂ (also referred to as NCM523), LiNi_{0.5}Co_{0.25}Mn_{0.25}O₂ (also referred to as NCM211), LiNi_{0.6}Co_{0.2}Mn_{0.2}O₂ (also referred to as NCM622), or LiNi_{0.8}Co_{0.1}Mn_{0.1}O₂ (also referred to as NCM811)), lithium nickel cobalt aluminum oxide (for example, LiNi_{0.85}Co_{0.15}Al_{0.05}O₂), and their modified compounds. Examples of olivine-structured lithium-containing phosphate may include but are not limited to at least one of lithium iron phosphate (for example, LiFePO₄ (also referred to as LFP)), a composite material of lithium iron phosphate and carbon, lithium manganese phosphate (for example, LiMnPO₄), a composite material of lithium manganese phosphate and carbon, lithium manganese iron phosphate, and a composite material of lithium manganese iron phosphate and carbon.

In some embodiments, the conductive agent may be a conductive agent known in the art for batteries. As an example, the conductive agent may include at least one of superconducting carbon, acetylene black, carbon black, Ketjen black, carbon dots, carbon nanotubes, graphene, and carbon nanofiber.

In some embodiments, the binder may be a binder known in the art for batteries. As an example, the binder may include polyvinylidene fluoride.

In some embodiments, the positive electrode plate may be prepared in the following method: the components for preparing the positive electrode plate, such as the positive electrode active material, the conductive agent, the binder, and any other components, are dispersed in a solvent (for example, N-methylpyrrolidone) to form a positive electrode slurry; and then the positive electrode slurry is applied onto the positive electrode current collector, followed by processes such as drying and cold pressing to obtain the positive electrode plate.

### [Negative electrode plate]

The negative electrode plate includes a negative electrode current collector and a negative electrode film layer disposed on at least one surface of the negative electrode current collector, where the negative electrode film layer includes a negative electrode active material.

As an example, the negative electrode current collector includes two opposite surfaces in its thickness direction, and the negative electrode film layer is disposed on either or both of the two opposite surfaces of the negative electrode current collector.

In some embodiments, the negative electrode current collector may be a metal foil or a composite current collector. For example, a copper foil may be used as the metal foil. The composite current collector may include a polymer material substrate and a metal layer formed on at least one surface of the polymer material substrate. The composite current collector may be formed by forming a metal material (for example, copper, copper alloy, nickel, nickel alloy, titanium, titanium alloy, silver, or silver alloy) on a polymer material substrate (for example, polypropylene (PP), polyethylene terephthalate (PET), polybutylene terephthalate (PBT), polystyrene (PS), or polyethylene (PE)).

In some embodiments, the negative electrode plate includes a negative electrode active material, where the negative electrode active material includes one or more of artificial graphite, natural graphite, hard carbon, soft carbon, mesocarbon microbeads, carbon fiber, carbon nanotubes, elemental silicon, a silicon-oxygen compound, a silicon-carbon composite, a silicon alloy, elemental tin, a tin-oxygen compound, and a titanium composite material.

In some embodiments, the titanium composite material includes lithium titanate.

The above negative electrode active materials each have an excellent specific capacity and a high specific surface area, enabling the battery to have a high energy density.

In some embodiments, the active specific surface area A of the negative electrode plate and the mass content W of the cyclic sulfate compound satisfy: 0.0002 cm²/g ≤ W × A ≤ 0.04 cm²/g, optionally 0.02 cm²/g ≤ W × A ≤ 0.1 cm²/g.

In this specification, the term "active specific surface area" refers to a ratio of the active surface area of the negative electrode plate to a mass of the negative electrode plate, which can be used to reflect the number of active sites during charging and discharging of the negative electrode plate. A larger active specific surface area of the negative electrode plate indicates more active sites in the negative electrode plate.

In this specification, the active specific surface area of the negative electrode plate can be tested using any known method. As an example, the active specific surface area of the negative electrode plate is calculated based on the Randles-Sevcik equation combined with the mass of the negative electrode plate. The negative electrode plate is used as a cathode, a metal lithium plate is used as the anode, ferrocene at a concentration of 50 mmol/L is added to an electrolyte (which is the same as an electrolyte used in a battery preparation method), and then a coin-type half-cell is assembled. Four parallel samples are respectively scanned at scanning speeds v of 0.1 mV/s, 0.3 mV/s, 0.5 mV/s, and 1 mV/s, to obtain cyclic voltammetry curves at different scanning speeds. The peak current ip of the cyclic voltammetry curve is extracted using EC-Lab software. A linear plot of the cyclic voltammetry curve ip versus the square root of the scanning speed v is made, with the square root of the scanning speed v as the horizontal coordinate and the peak current ip as the vertical coordinate, to obtain the slope K.

According to the Randles-Sevcik equation, the slope is known, where n represents the number of electron transfers, related to the type of a probe molecule, here taken as 1; c represents the concentration of ferrocene, taken as 50 mmol/L; D is a diffusion coefficient of ferrocene, taken as 2.1 × 10⁻⁶ cm²/s; then, the active surface area A of the negative electrode plate is equal to K / (2.69 × 10⁵ × n^{2/3} cD^{1/2}), and the ratio of the active surface area A of the negative electrode plate to the mass m of the negative electrode plate is the active specific surface area of the negative electrode plate.

In some embodiments, the product of the active specific surface area A of the negative electrode plate and the mass content W of the cyclic sulfate compound is optionally 0.0002 cm²/g, 0.0005 cm²/g, 0.002 cm²/g, 0.005 cm²/g, 0.02 cm²/g, 0.022 cm²/g, 0.024 cm²/g, 0.025 cm²/g, 0.026 cm²/g, 0.028 cm²/g, 0.03 cm²/g, 0.032 cm²/g, 0.034 cm²/g, 0.035 cm²/g, 0.036 cm²/g, 0.038 cm²/g, 0.04 cm²/g, or a value within a range defined by any two of the above values.

When the active specific surface area A of the negative electrode plate and the mass content W of the cyclic sulfate compound satisfy the above relationship, a stable SEI film can be formed, and the rate performance and cycling performance of the battery are balanced, fully utilizing the battery capacity.

In some embodiments, the active specific surface area A of the negative electrode plate satisfies: A ≤ 20 cm²/g, optionally 5 cm²/g to 15 cm²/g.

In some embodiments, the active specific surface area A of the negative electrode plate is optionally 1 cm²/g, 2 cm²/g, 4 cm²/g, 5 cm²/g, 6 cm²/g, 8 cm²/g, 10 cm²/g, 11 cm²/g, 12 cm²/g, 13 cm²/g, 14 cm²/g, 15 cm²/g, or a value within a range defined by any two of the above values.

Controlling the active specific surface area A of the negative electrode plate within an appropriate range can avoid or reduce increased battery polarization due to an excessively small active specific surface area of the negative electrode plate so as not to affect the utilization of the battery capacity, and can also avoid or reduce increased interface side reactions due to an excessively large active specific surface area of the negative electrode plate so as not to affect the cycling performance and storage performance of the battery. An active specific surface area A within an appropriate range can balance the cycling performance, storage performance, and capacity of the battery.

In some embodiments, the negative electrode film layer further optionally includes a binder. The binder may be selected from at least one of styrene-butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), and carboxymethyl chitosan (CMCS).

In some embodiments, the negative electrode film layer further optionally includes a conductive agent. The conductive agent may be selected from at least one of superconducting carbon, acetylene black, carbon black, Ketjen black, carbon dots, carbon nanotubes, graphene, and carbon nanofiber.

In some embodiments, the negative electrode film layer further optionally includes other additives, such as a thickener (for example, sodium carboxymethyl cellulose (CMC-Na)).

In some embodiments, the negative electrode plate may be prepared in the following method: the components for preparing the negative electrode plate, such as the negative electrode active material, the conductive agent, the binder, and any other components, are dispersed in a solvent (for example, deionized water) to form a negative electrode slurry; and then the negative electrode slurry is applied onto the negative electrode current collector, followed by processes such as drying and cold pressing to obtain the negative electrode plate.

### [Separator]

In some embodiments, the secondary battery further includes a separator. In this application, the separator may be any well-known porous structure separator with good chemical and mechanical stability.

In some embodiments, the material of the separator may be selected from at least one of glass fiber, non-woven fabric, polyethylene, polypropylene, and polyvinylidene fluoride. The separator may be a single-layer film or a multilayer composite film. When the separator is a multilayer composite film, the materials of the layers may be the same or different.

In some embodiments, the positive electrode plate, negative electrode plate, and separator may be formed into an electrode assembly through a winding process or a lamination process.

In some embodiments, the secondary battery may include an outer package. The outer package may be used to encapsulate the above electrode assembly and electrolyte.

In some embodiments, the outer package of the secondary battery may be a hard shell, for example, a hard plastic shell, an aluminum shell, or a steel shell. The outer package of the secondary battery may alternatively be a soft pack, for example, a soft pouch. The material of the soft pack may be plastic. As the plastic, polypropylene, polybutylene terephthalate, polybutylene succinate, and the like may be listed.

### [Secondary battery]

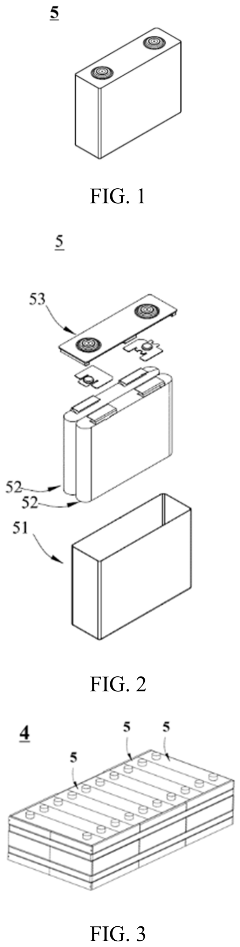

In this application, the shape of the secondary battery includes but is not limited to a cylindrical shape, a rectangular shape, or any other shapes. For example, FIG. 1 shows a secondary battery 5 with a rectangular structure as an example. The secondary battery may alternatively be a sodium-ion secondary battery, a magnesium-ion secondary battery, or a potassium-ion secondary battery.

In some embodiments, the secondary battery includes the electrolyte provided in this application. In some embodiments, the secondary battery includes the negative electrode plate provided in this application.

In some embodiments, referring to FIG. 2, the outer package may include a housing 51 and a cover plate 53. The housing 51 may include a bottom plate and side plates connected to the bottom plate, with the bottom plate and side plates enclosing an accommodating cavity. The housing 51 has an opening communicating with the accommodating cavity, and the cover plate 53 can cover the opening to seal the accommodating cavity. The positive electrode plate, the negative electrode plate, and the separator may be formed into an electrode assembly 52 through winding or lamination. The electrode assembly 52 is encapsulated in the accommodating cavity. The electrolyte infiltrates the electrode assembly 52. The secondary battery 5 may include one or more electrode assemblies 52, and persons skilled in the art can make choices based on specific actual requirements.

### [Battery module]

In some embodiments, the secondary battery may be assembled into a battery module, and the battery module may include one or more secondary batteries. The specific quantity may be chosen by persons skilled in the art according to use and capacity of the battery module.

FIG. 3 shows a battery module 4 as an example. Referring to FIG. 3, in the battery module 4, multiple secondary batteries 5 may be arranged sequentially along a length direction of the battery module 4. Certainly, the secondary batteries 5 may alternatively be arranged in any other manner. Further, the multiple secondary batteries 5 may be fixed by fasteners.

Optionally, the battery module 4 may further include an enclosure with an accommodating space, where the multiple secondary batteries 5 are accommodated in the accommodating space.

### [Battery pack]

In some embodiments, the above battery modules can be assembled into a battery pack, and of the battery pack may include one or more battery modules. The specific quantity may be chosen by persons skilled in the art according to use and capacity of the battery pack.

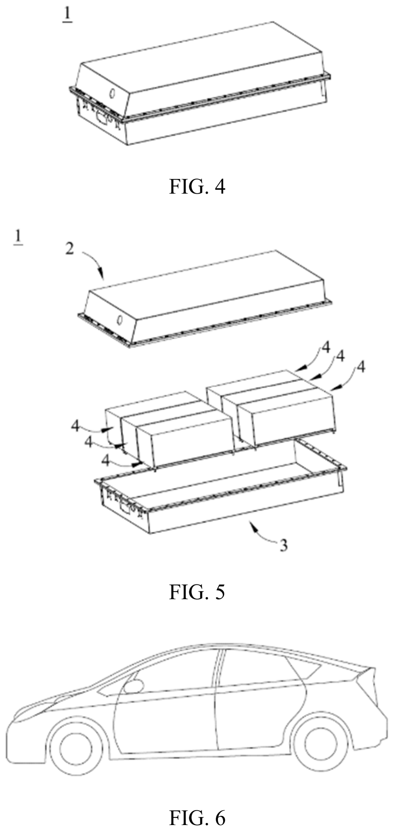

FIG. 4 and FIG. 5 show a battery pack 1 as an example. Referring to FIG. 4 and FIG. 5, the battery pack 1 may include a battery box and multiple battery modules 4 disposed in the battery box. The battery box includes an upper box body 2 and a lower box body 3, where the upper box body 2 can cover the lower box body 3 to form a closed space for accommodating the battery modules 4. The multiple battery modules 4 may be arranged in the battery box in any manner.

### [Electric apparatus]

An embodiment of this application provides an electric apparatus including at least one of the secondary battery according to any embodiment, the battery module according to any embodiment, or the battery pack according to any embodiment.

The electric apparatus includes at least one of the secondary battery, battery module, or battery pack provided in this application. The secondary battery, battery module, or battery pack may be used as a power source for the electric apparatus or as an energy storage unit for the electric apparatus. The electric apparatus may include but is not limited to a mobile device (for example, a mobile phone or a notebook computer), an electric vehicle (for example, a battery electric vehicle, a hybrid electric vehicle, a plug-in hybrid electric vehicle, an electric bicycle, an electric scooter, an electric golf cart, or an electric truck), an electric train, a ship, a satellite system, or an energy storage system.

As an electric apparatus, the secondary battery, battery module, or battery pack can be selected based on its usage requirements.

FIG. 6 shows an electric apparatus as an example. The electric apparatus is a battery electric vehicle, a hybrid electric vehicle, or a plug-in hybrid electric vehicle. To satisfy requirements of the electric apparatus for high power and high energy density of a secondary battery, a battery pack or a battery module may be used.

As another example, the apparatus may be a mobile phone, a tablet computer, a notebook computer, or the like. Such apparatus is typically required to be light and thin and may use a secondary battery as its power source.

### Examples

The following describes examples of this application. The examples described below are illustrative and only used to explain this application, and cannot be construed as limitations on this application. Examples whose technical solutions or conditions are not specified are made in accordance with technical solutions or conditions described in literature in the field or made in accordance with product instructions. The reagents or instruments used are all conventional products that are commercially available if no manufacturer is indicated.

### I. Preparation method

### Example 1

### (1) Preparation of cyclic sulfate compound

364.3 g (2 mol) of galactitol was added to a 4 L three-neck flask, and stirring was started. 784.5 g (6.6 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

140 g (0.4 mol) of the above intermediate product was added to a 4 L three-neck flask, and then 1000 mL of acetonitrile and 110 mg of ruthenium trichloride trihydrate catalyst were added. After nitrogen replacement, the system was cooled to 20°C, and stirring was started. 1500 g of a 20% sodium hypochlorite aqueous solution was added dropwise in 1 h, with the reaction temperature controlled at 15°C. After the addition was complete, the mixture was stirred at 15°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound I-1.

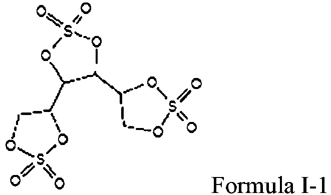

(2) Preparation of positive electrode plate: A 2.5wt% polyvinylidene fluoride binder was fully dissolved in N-methylpyrrolidone (NMP), followed by the addition of 2.0wt% Super P, 1.0wt% carbon nanotubes, and 94.5wt% positive electrode active material lithium iron phosphate (LiFePO₄). The mixture was stirred and mixed well to form a positive electrode slurry. The slurry was evenly applied onto a surface of a current collector aluminum foil and then transferred to a vacuum drying oven for complete drying. The dried electrode plate was rolled and punched to obtain the positive electrode plate.

### (3) Preparation of negative electrode plate

Artificial graphite as an active material, carbon black as a conductive agent, styrene-butadiene rubber (SBR) as a binder, and sodium carboxymethyl cellulose (CMC) as a thickener were dissolved in deionized water as a solvent at a weight ratio of 96.2:0.8:0.8:1.2. The mixture was mixed well to prepare a negative electrode slurry. The negative electrode slurry was applied once or multiple times evenly onto a negative electrode current collector copper foil, followed by drying, cold pressing, and slitting to obtain the negative electrode plate. An active specific surface area of the negative electrode plate was 8 cm²/g.

### (4) Electrolyte

In an argon atmosphere glove box (H₂O < 0.1 ppm, O₂ < 0.1 ppm), lithium salt lithium hexafluorophosphate LiPF₆, the cyclic sulfate compound with the structure represented by Formula 1-1, and a metal ion additive sodium hexafluorophosphate NaPF₆ were dissolved in a mixed system of organic solvents ethylene carbonate/dimethyl carbonate/ethyl methyl carbonate (with a mass ratio of EC:DMC:EMC being 1:1:1). The mixture was stirred well to obtain an electrolyte with a lithium salt at a concentration of 1 mol/L.

### (5) Separator

A polypropylene film was used as the separator.

### (6) Battery preparation

The positive electrode plate, the separator, and the negative electrode plate were stacked sequentially, so that the separator was located between the positive and negative electrode plates to provide isolation, and then the resulting stack was wound to obtain an electrode assembly. The electrode assembly was placed in a battery housing, dried, and injected with the electrolyte, followed by processes such as standing, hot and cold pressing, formation, shaping, and capacity testing to obtain a lithium battery product of Example 1.

### Example 2

The preparation method of the battery of Example 2 was similar to the preparation method of the battery of Example 1, but the preparation method of the cyclic sulfate compound was adjusted. The specific preparation method was described below.

392.4 g (2 mol) of solid 1,2,3,4,5,6-heptanhexol was added to a 4 L three-neck flask, and stirring was started. 784.5 g (6.6 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

140 g (0.4 mol) of the intermediate product 1 was added to a 4 L three-neck flask, and then 1000 mL of acetonitrile and 110 mg of ruthenium trichloride trihydrate catalyst were added. After nitrogen replacement, the system was cooled to 20°C, and stirring was started. 1500 g of a 20% sodium hypochlorite aqueous solution was added dropwise in 1 h, with the reaction temperature controlled at 15°C. After the addition was complete, the mixture was stirred at 15°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound 1-2.

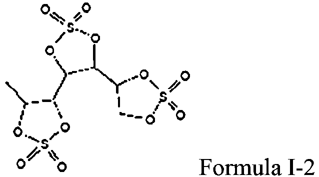

### Example 3

The preparation method of the battery of Example 3 was similar to the preparation method of the battery of Example 1, but the preparation method of the cyclic sulfate compound was adjusted. The specific preparation method was described below.

484 g (2 mol) of solid octitol was added to a 2 L three-neck flask, and stirring was started. 1046 g (8.8 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

183.2 g (0.4 mol) of the intermediate product was added to a 4 L three-neck flask, and then 1000 mL of acetonitrile and 150 mg of ruthenium trichloride trihydrate catalyst were added. After nitrogen replacement, the system was cooled to 20°C, and stirring was started. 2000 g of a 20% sodium hypochlorite aqueous solution was added dropwise in 1 h, with the reaction temperature controlled at 10-20°C. After the addition was complete, the mixture was stirred at 10-20°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound I-5.

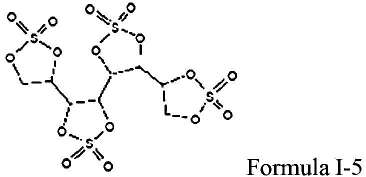

### Example 4

The preparation method of the battery of Example 4 was similar to the preparation method of the battery of Example 1, but the preparation method of the cyclic sulfate compound was adjusted. The specific preparation method was described below.

300 g (2 mol) of solid 1,6-dideoxygalactitol was added to a 3 L three-neck flask, and stirring was started. 523 g (4.4 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

184.2 g (0.8 mol) of the above intermediate product was added to a 3 L three-neck flask, and then 1000 mL of acetonitrile and 80 mg of ruthenium trichloride trihydrate catalyst were added. After nitrogen replacement, the system was cooled to 20°C, and stirring was started. 2000 g of a 20% sodium hypochlorite aqueous solution was added dropwise in 1 h, with the reaction temperature controlled at 15°C. After the addition was complete, the mixture was stirred at 15°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a white powder solid which was a compound 1-6. 1H-NMR, CD3CN, δ ppm 5.42-5.39 (m, 2H), 5.36-5.34 (m, 2H), 1.67-1.65 (d, 6H).

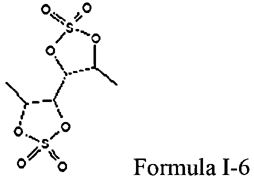

### Example 5

The preparation method of the battery of Example 5 was similar to the preparation method of the battery of Example 1, but the preparation method of the cyclic sulfate compound was adjusted. The specific preparation method was described below.

356.5 g (2 mol) of solid 3,4,5,6-octanetetraol was added to a 2 L three-neck flask, and stirring was started. 523 g (4.4 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

216.2 g (0.8 mol) of the above intermediate product was added a 3 L three-neck flask, and then 1000 mL of acetonitrile and 80 mg of ruthenium trichloride trihydrate catalyst were added. After nitrogen replacement, the system was cooled to 20°C, and stirring was started. 2000 g of a 20% sodium hypochlorite aqueous solution was added dropwise in 1 h, with the reaction temperature controlled at 15°C. After the addition was complete, the mixture was stirred at 15°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound I-7.

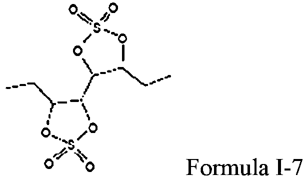

### Example 6

The preparation method of the battery of Example 6 was similar to the preparation method of the battery of Example 1, but the preparation method of the cyclic sulfate compound was adjusted. The specific preparation method was described below.

328.4 g (2 mol) of solid 2,3,4,5-heptanetetraol was added to a 2 L three-neck flask, and stirring was started. 523 g (4.4 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

205 g (0.8 mol) of the above intermediate product was added to a 3 L three-neck flask, and then 1000 mL of acetonitrile was added, and the mixture was stirred until the solid was completely dissolved. 80 mg of ruthenium trichloride trihydrate catalyst was added. After nitrogen replacement, the system was cooled to 20°C, and stirring was started. 2000 g of a 20% sodium hypochlorite aqueous solution was added dropwise in 1 h, with the reaction temperature controlled at 10-20°C. After the addition was complete, the mixture was stirred at 10-20°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound I-8.

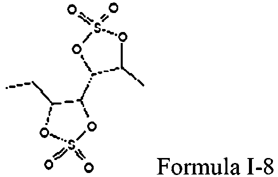

### Examples 7 to 29

The preparation methods of the batteries of Examples 7 to 29 were similarly to the preparation method of the battery of Example 4, but the mass content of the cyclic sulfate compound, the type and mass content of the metal ions, or the type of the negative electrode active material were adjusted. Specific parameters are shown in Table 1.

### Example 30

### (1) Preparation of cyclic sulfate compound

364.3 g (2 mol) of galactitol was added to a 4 L three-neck flask, and stirring was started. 784.5 g (6.6 mol) of thionyl chloride was added dropwise to the three-neck flask, with the temperature controlled at approximately 15°C during the addition. After the addition was complete, the reaction was maintained at 45°C for 4 h, resulting in the precipitation of a large amount of pasty solid from the reaction solution. After cooling was performed, 1 L of deionized water was slowly added dropwise, and the reaction system was rapidly stirred and dispersed. The solid obtained by filtration was pulped and washed multiple times with deionized water until the pH was neutral. The filter cake was dried under reduced pressure at 60°C to obtain an intermediate product.

140 g (0.4 mol) of the above intermediate product was added to a 4 L three-neck flask, and then 1000 mL of acetonitrile and 110 mg of ruthenium trichloride trihydrate catalyst were added. After nitrogen replacement, the system was cooled to 20°C, and stirring was started. 1500 g of a 20% sodium hypochlorite aqueous solution was added dropwise in 1 h, with the reaction temperature controlled at 15°C. After the addition was complete, the mixture was stirred at 15°C for 10 min, and solution separation was performed. An organic phase was quenched with a sodium sulfite aqueous solution until the starch-potassium iodide test paper no longer turned blue. Solution separation was performed again. An organic layer was concentrated, and acetonitrile was crystallized, to obtain a compound I-1.

### (2) Preparation of positive electrode plate

A positive electrode active material NaFePO₄, a conductive agent acetylene black, and a binder polyvinylidene fluoride (PVDF) were stirred and mixed well in an N-methylpyrrolidone solvent system at a mass ratio of 80:10:10 to obtain a positive electrode slurry. The slurry was evenly applied onto a surface of a current collector aluminum foil and then transferred to a vacuum drying oven for complete drying. The dried electrode plate was rolled and punched to obtain the positive electrode plate.

### (3) Preparation of negative electrode plate

A negative electrode active material hard carbon, a conductive agent acetylene black, and a binder polyacrylic acid were stirred and mixed well in a deionized water solvent system at a mass ratio of 88:2:10 to prepare a negative electrode slurry. The negative electrode slurry was applied once or multiple times evenly onto a negative electrode current collector copper foil, followed by drying, cold pressing, and slitting to obtain the negative electrode plate. The active specific surface area of the negative electrode plate was 8 cm²/g.

### (4) Electrolyte

In an argon atmosphere glove box (H₂O < 0.1 ppm, O₂ < 0.1 ppm), a sodium salt sodium hexafluorophosphate NaPF₆, the cyclic sulfate compound with the structure represented by Formula I-1, and a metal ion additive lithium hexafluorophosphate LiPF₆ were dissolved in a mixed system of organic solvents ethylene carbonate/dimethyl carbonate/ethyl methyl carbonate (with a mass ratio of EC:DMC:EMC being 1:1:1). The mixture was stirred well to obtain an electrolyte with a sodium salt at a concentration of 1 mol/L.

### (5) Separator

A polypropylene film was used as the separator.

### (6) Battery preparation

The positive electrode plate, the separator, and the negative electrode plate were stacked sequentially, so that the separator was located between the positive and negative electrode plates to provide isolation, and then the resulting stack was wound to obtain an electrode assembly. The electrode assembly was placed in a battery housing, dried, and injected with the electrolyte, followed by processes such as standing, hot and cold pressing, formation, shaping, and capacity testing to obtain a sodium battery product of Example 33.

### Examples 31 to 41

The preparation methods of the batteries of Examples 31 to 41 were similar to the preparation method of the battery of Example 30, but the type and mass content of the cyclic sulfate compound and the type of the metal ions were adjusted. Specific parameters are shown in Table 1.

### Examples 42 to 44

The preparation methods of the batteries of Examples 42 to 44 were similar to the preparation method of the battery of Example 4, but the active specific surface area of the negative electrode plate was adjusted. Specific parameters are shown in Table 1.

### Comparative example 1

The preparation method of the battery of Comparative example 1 was similar to the preparation method of the battery of Example 1, but the electrolyte did not contain a metal ion additive. Specific parameters are shown in Table 1.

### Comparative example 2

The preparation method of the battery of Comparative example 2 was similar to the preparation method of the battery of Example 33, but the electrolyte did not contain a metal ion additive. Specific parameters are shown in Table 1.

### Comparative example 3

The preparation method of the battery of Comparative example 3 was similar to the preparation method of the battery of Example 1, but the electrolyte did not contain a cyclic sulfate compound. Specific parameters are shown in Table 1.

### Comparative example 4

The preparation method of the battery of Comparative example 4 was similar to the preparation method of the battery of Example 33, but the electrolyte did not contain a cyclic sulfate compound. Specific parameters are shown in Table 1.

### Comparative example 5

The preparation method of the battery of Comparative example 5 was similar to the preparation method of the battery of Example 1, but the sulfate compound used was a commercial compound, ethylene bis(sulfate) (CAS No.1431298-10-0, commercially available), where the structural formula of ethylene bis(sulfate) was shown as Formula III. Specific parameters are shown in Table 1.

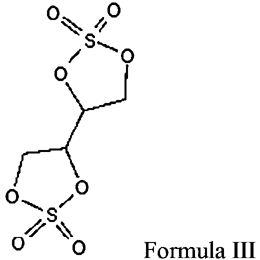

### Comparative example 6

The preparation method of the battery of Comparative example 6 was similar to the preparation method of the battery of Comparative example 5, but the type of the electrolytic salt in the electrolyte was adjusted. Specific parameters are shown in Table 1.

### II. Performance test

### 1. Performance test of negative electrode plate

### (1) Test of active specific surface area

The negative electrode plate prepared in each of the examples and comparative examples was used as a cathode, a metal lithium plate was used as n anode, ferrocene at a concentration of 50 mmol/L was added to an electrolyte (which was the same as an electrolyte used in a battery preparation method), and then a coin-type half-cell was assembled. Four parallel samples were respectively scanned at scanning speeds v of 0.1 mV/s, 0.3 mV/s, 0.5 mV/s, and 1 mV/s, to obtain cyclic voltammetry curves at different scanning speeds. The peak current ip of the cyclic voltammetry curve was extracted using EC-Lab software. A linear plot of the cyclic voltammetry curve ip versus the square root of the scanning speed v was made, with the square root of the scanning speed v as the horizontal coordinate and the peak current ip as the vertical coordinate, to obtain the slope K.

According to the Randles-Sevcik equation, the slope was known, where n represented the number of electron transfers, related to the type of a probe molecule, here taken as 1; c represented the concentration of ferrocene, taken as 50 mmol/L; D was the diffusion coefficient of ferrocene, taken as 2.1 × 10⁻⁶ cm²/s; then, the active surface area A of the negative electrode plate was equal to K / (2.69 × 10⁵ × n^{2/3} cD^{1/2}), and the ratio of the active surface area A of the negative electrode plate to the mass m of the negative electrode plate was the active specific surface area of the negative electrode plate.

### 2. Battery performance test

### (1) Test of the number of cycles to capacity retention rate of 80% at 25°C

A process of the test of the number of cycles to capacity retention rate of 80% was conducted as follows: At 25°C, the prepared battery was charged at a constant current of 1C to 3.65 V, then charged at a constant voltage of 3.65 V until the current dropped to 0.1C, and discharged at 1C to 2.5 V. The resulting capacity was recorded as an initial capacity (C0). The above steps were repeated for the same battery, and a discharge capacity (Cn) of the battery after the n-th cycle was recorded. A capacity retention rate Pn after each cycle was equal to Cn / C0 × 100%. The number of cycles was recorded when Pn dropped to 80%.

### (2) Test of rate performance

At 25°C, the prepared battery was charged at a constant current of 0.5C to 3.65 V, then charged at a constant voltage of 3.65 V until the current dropped below 0.05C, and left standing for 5 minutes. The battery was then discharged to 2.5 V at different rates of 0.2C, 1.0C, 1.5C, and 2.0C. The battery was left standing for 5 minutes at the end of each charge, and a discharge capacity of the battery was recorded. A discharge capacity obtained when the battery was discharged at the rate of 0.2C was used as the reference. Five parallel samples were prepared for each group, and the average was taken. Discharge capacity ratios of the battery at different discharge rates were calculated using the formula described below.

Discharge capacity ratio of the battery at a rate (%) = (a discharge capacity at a corresponding rate / the discharge capacity at the rate of 0.2C) × 100%.

### III. Analysis of test results of Examples and Comparative examples

The batteries in the examples and comparative examples were prepared according to the foregoing methods, and performance parameters were measured. Results are shown in Table 1 and Table 2.

**Table 1**

| No. | Electrolyte | | | | | Negative electrode plate | |
|---|---|---|---|---|---|---|---|
| | Cyclic sulfate compound | | Metal ions in metal ion additive | | Electrolytic salt | Negative electrode active material | Active specific surface area (cm²/g) |
| | Material | Mass content (%) | Metal ions | Mass content (ppm) | | | |
| Example 1 | Formula I-1 | 2.5 | Sodium ions | 300 | Lithium hexafluorophosphate | Artificial graphite | 8 |
| Example 2 | Formula I-2 | 2.5 | Sodium ions | 300 | Lithium hexafluorophosphate | Artificial graphite | 8 |
| Example 3 | Formula I-5 | 2.5 | Sodium ions | 300 | Lithium hexafluorophosphate | Artificial graphite | 8 |
| Example 4 | Formula I-6 | 2.5 | Sodium ions | 300 | Lithium hexafluorophosphate | Artificial graphite | 8 |
| Example 5 | Formula I-7 | 2.5 | Sodium ions | 300 | Lithium hexafluorophosphate | Artificial graphite | 8 |
| Example 6 | Formula I-8 | 2.5 | Sodium ions | 300 | Lithium hexafluorophosphate | Artificial graphite | 8 |
| Example 7 | Formula I-6 | 0.001 | Sodium ions | 300 | Lithium hexafluorophosphate | Artificial graphite | 8 |
| Example 8 | Formula I-6 | 0.1 | Sodium ions | 300 | Lithium hexafluorophosphate | Artificial graphite | 8 |
| Example 9 | Formula I-6 | 5 | Sodium ions | 300 | Lithium hexafluorophosphate | Artificial graphite | 8 |
| Example 10 | Formula I-6 | 20 | Sodium ions | 300 | Lithium hexafluorophosphate | Artificial graphite | 8 |
| Example 11 | Formula I-6 | 2.5 | Sodium ions | 30 | Lithium hexafluorophosphate | Artificial graphite | 8 |
| Example 12 | Formula I-6 | 2.5 | Sodium ions | 50 | Lithium hexafluorophosphate | Artificial graphite | 8 |
| Example 13 | Formula I-6 | 2.5 | Sodium ions | 1000 | Lithium hexafluorophosphate | Artificial graphite | 8 |
| Example 14 | Formula I-6 | 2.5 | Sodium ions | 2000 | Lithium hexafluorophosphate | Artificial graphite | 8 |
| Example 15 | Formula I-6 | 2.5 | Sodium ions | 3000 | Lithium hexafluorophosphate | Artificial graphite | 8 |
| Example 16 | Formula I-6 | 2.5 | Aluminum ions | 15 | Lithium hexafluorophosphate | Artificial graphite | 8 |
| Example 17 | Formula I-6 | 2.5 | Aluminum ions | 50 | Lithium hexafluorophosphate | Artificial graphite | 8 |
| Example 18 | Formula I-6 | 2.5 | Aluminum ions | 100 | Lithium hexafluorophosphate | Artificial graphite | 8 |
| Example 19 | Formula I-6 | 2.5 | Aluminum ions | 200 | Lithium hexafluorophosphate | Artificial graphite | 8 |
| Example 20 | Formula I-6 | 2.5 | Aluminum ions | 300 | Lithium hexafluorophosphate | Artificial graphite | 8 |
| Example 21 | Formula I-6 | 2.5 | Magnesium ions | 13 | Lithium hexafluorophosphate | Artificial graphite | 8 |
| Example 22 | Formula I-6 | 2.5 | Magnesium ions | 50 | Lithium hexafluorophosphate | Artificial graphite | 8 |
| Example 23 | Formula I-6 | 2.5 | Magnesium ions | 100 | Lithium hexafluorophosphate | Artificial graphite | 8 |
| Example 24 | Formula I-6 | 2.5 | Magnesium ions | 150 | Lithium hexafluorophosphate | Artificial graphite | 8 |
| Example 25 | Formula I-6 | 2.5 | Magnesium ions | 400 | Lithium hexafluorophosphate | Artificial graphite | 8 |
| Example 26 | Formula I-6 | 2.5 | Magnesium ions | 500 | Lithium hexafluorophosphate | Artificial graphite | 8 |
| Example 27 | Formula I-6 | 2.5 | Copper ions | 50 | Lithium hexafluorophosphate | Artificial graphite | 8 |
| Example 28 | Formula I-6 | 2.5 | Calcium ions | 50 | Lithium hexafluorophosphate | Artificial graphite | 8 |
| Example 29 | Formula I-6 | 2.5 | Sodium ions | 300 | Lithium hexafluorophosphate | Hard carbon | 8 |
| Example 30 | Formula I-1 | 2.5 | Lithium ions | 300 | Sodium hexafluorophosphate | Hard carbon | 8 |
| Example 31 | Formula I-2 | 2.5 | Lithium ions | 300 | Sodium hexafluorophosphate | Hard carbon | 8 |
| Example 32 | Formula I-5 | 2.5 | Lithium ions | 300 | Sodium hexafluorophosphate | Hard carbon | 8 |
| Example 33 | Formula I-6 | 2.5 | Lithium ions | 300 | Sodium hexafluorophosphate | Hard carbon | 8 |
| Example 34 | Formula I-7 | 2.5 | Lithium ions | 300 | Sodium hexafluorophosphate | Hard carbon | 8 |
| Example 35 | Formula I-8 | 2.5 | Lithium ions | 300 | Sodium hexafluorophosphate | Hard carbon | 8 |
| Example 36 | Formula I-6 | 0.001 | Lithium ions | 300 | Sodium hexafluorophosphate | Hard carbon | 8 |
| Example 37 | Formula I-6 | 0.1 | Lithium ions | 300 | Sodium hexafluorophosphate | Hard carbon | 8 |
| Example 38 | Formula I-6 | 5 | Lithium ions | 300 | Sodium hexafluorophosphate | Hard carbon | 8 |
| Example 39 | Formula I-6 | 20 | Lithium ions | 300 | Sodium hexafluorophosphate | Hard carbon | 8 |
| Example 40 | Formula I-6 | 2.5 | Aluminum ions | 50 | Sodium hexafluorophosphate | Hard carbon | 8 |
| Example 41 | Formula I-6 | 2.5 | Magnesium ions | 50 | Sodium hexafluorophosphate | Hard carbon | 8 |
| Example 42 | Formula I-6 | 2.5 | Sodium ions | 300 | Lithium hexafluorophosphate | Artificial graphite | 5 |
| Example 43 | Formula I-6 | 2.5 | Sodium ions | 300 | Lithium hexafluorophosphate | Artificial graphite | 15 |
| Example 44 | Formula I-6 | 2.5 | Sodium ions | 300 | Lithium hexafluorophosphate | Artificial graphite | 20 |
| Comparative example 1 | Formula I-6 | 2.5 | / | / | Lithium hexafluorophosphate | Artificial graphite | 8 |
| Comparative example 2 | Formula I-6 | 2.5 | / | / | Sodium hexafluorophosphate | Hard carbon | 8 |
| Comparative example 3 | / | / | Sodium ions | 300 | Lithium hexafluorophosphate | Artificial graphite | 8 |
| Comparative example 4 | / | / | Lithium ions | 300 | Sodium hexafluorophosphate | Hard carbon | 8 |
| Comparative example 5 | Ethylene bis(sulfate) | 2.5 | Sodium ions | 300 | Lithium hexafluorophosphate | Artificial graphite | 8 |
| Comparative example 6 | Ethylene bis(sulfate) | 2.5 | Lithium ions | 300 | Sodium hexafluorophosphate | Hard carbon | 8 |

**Table 2**

| No. | Battery | | | | |
|---|---|---|---|---|---|
| | Cycles to capacity retention rate of 80% (cycles) | Ratios of discharge capacities at different rates to discharge capacity at 0.2C (%) | | | |
| | | 0.2C | 1C | 1.5C | 2C |
| Example 1 | 2643 | 100% | 95.1% | 92.5% | 82.7% |
| Example 2 | 2554 | 100% | 94.7% | 92.1% | 82.3% |
| Example 3 | 2601 | 100% | 94.1% | 92.5% | 81.8% |
| Example 4 | 2789 | 100% | 95.7% | 93.2% | 83.5% |
| Example 5 | 2591 | 100% | 94.8% | 92.4% | 82.5% |
| Example 6 | 2576 | 100% | 95.0% | 92.1% | 82.0% |
| Example 7 | 2287 | 100% | 94.9% | 91.9% | 81.7% |
| Example 8 | 2432 | 100% | 95.2% | 92.3% | 82.5% |
| Example 9 | 2534 | 100% | 95.5% | 92.5% | 82.7% |
| Example 10 | 2411 | 100% | 95.3% | 92.4% | 82.4% |
| Example 11 | 2518 | 100% | 94.8% | 91.8% | 82.0% |
| Example 12 | 2578 | 100% | 95.1% | 92.0% | 82.2% |
| Example 13 | 2611 | 100% | 95.3% | 92.3% | 82.9% |
| Example 14 | 2641 | 100% | 95.2% | 92.2% | 82.7% |
| Example 15 | 2598 | 100% | 95.0% | 92.0% | 82.5% |
| Example 16 | 2477 | 100% | 95.1% | 92.2% | 82.1% |
| Example 17 | 2658 | 100% | 95.5% | 92.6% | 82.7% |
| Example 18 | 2627 | 100% | 95.6% | 92.5% | 82.6% |
| Example 19 | 2591 | 100% | 95.3% | 92.3% | 82.4% |
| Example 20 | 2587 | 100% | 95.1% | 92.1% | 82.9% |
| Example 21 | 2456 | 100% | 95.0% | 92.0% | 82.4% |
| Example 22 | 2672 | 100% | 95.4% | 92.5% | 82.7% |
| Example 23 | 2643 | 100% | 95.5% | 92.4% | 82.5% |
| Example 24 | 2587 | 100% | 95.6% | 92.1% | 82.5% |
| Example 25 | 2594 | 100% | 95.2% | 92.3% | 82.3% |
| Example 26 | 2531 | 100% | 95.1% | 92.1% | 82.0% |
| Example 27 | 2567 | 100% | 95.3% | 92.8% | 82.6% |
| Example 28 | 2611 | 100% | 95.2% | 93.0% | 82.4% |
| Example 29 | 2311 | 100% | 95.8% | 93.0% | 83.4% |
| Example 30 | 659 | 100% | 96.7% | 93.7% | 85.7% |
| Example 31 | 598 | 100% | 95.5% | 92.8% | 84.2% |
| Example 32 | 641 | 100% | 96.4% | 93.3% | 85.8% |
| Example 33 | 711 | 100% | 97.1% | 94.1% | 86.1% |
| Example 34 | 603 | 100% | 96.2% | 93.6% | 85.3% |
| Example 35 | 623 | 100% | 96.5% | 93.5% | 85.4% |
| Example 36 | 387 | 100% | 96.2% | 92.9% | 84.2% |
| Example 37 | 552 | 100% | 96.7% | 93.1% | 85.2% |
| Example 38 | 698 | 100% | 96.8% | 93.2% | 85.7% |
| Example 39 | 672 | 100% | 96.2% | 93.0% | 85.4% |
| Example 40 | 668 | 100% | 96.7% | 93.4% | 85.7% |
| Example 41 | 691 | 100% | 96.9% | 93.6% | 85.5% |
| Example 42 | 2611 | 100% | 94.8% | 92.1% | 82.7% |
| Example 43 | 2548 | 100% | 95.8% | 93.3% | 83.7% |
| Example 44 | 2011 | 100% | 95.9% | 93.4% | 83.7% |
| Comparative example 1 | 2317 | 100% | 94.7% | 91.7% | 81.1% |
| Comparative example 2 | 657 | 100% | 95.1% | 92.1% | 83.5% |
| Comparative example 3 | 1741 | 100% | 93.5% | 90.2% | 80.5% |
| Comparative example 4 | 365 | 100% | 94.7% | 91.6% | 82.3% |
| Comparative example 5 | 1871 | 100% | 94.5% | 91.4% | 81.6% |
| Comparative example 6 | 387 | 100% | 95.1% | 91.9% | 83.4% |

It can be seen from the above results that the electrolytes in Examples 1 to 41 each include a cyclic sulfate compound represented by Formula I and a metal ion additive, where in Formula I, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and R₁, R₂, R₃, and R₄ are not all selected from a hydrogen atom; and
in Formula II, R₅ and R₆ are each independently selected from a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and a wavy line indicates a bonding position.

It can be seen from the comparison of Examples 1 to 29 with Comparative examples 1 and 3 and the comparison of Examples 30 to 41 with Comparative examples 2 and 4 that compared to an electrolyte including only the cyclic sulfate compound represented by Formula I or the metal ion additive, the electrolyte of this application includes both the cyclic sulfate compound represented by Formula I and the metal ion additive. This is conducive to increasing the ratios of discharge capacities of the battery at the rates of 1.5C and 2C to the discharge capacity at 0.2C, thereby improving the rate performance of the battery.

It can be seen from the comparison of Examples 1 to 29 with Comparative example 5 and the comparison of Examples 30 to 41 with Comparative example 6 that compared to an electrolyte including a cyclic sulfate compound represented by Formula III and a metal ion additive, the electrolyte of this application includes both the cyclic sulfate compound represented by Formula I and the metal ion additive. This is conducive to increasing the number of cycles to capacity retention rate of 80% of the battery and ratios of discharge capacities at the rates of 1.5C and 2C to the discharge capacity at 0.2C, thereby improving the cycling performance and rate performance of the battery.

It can be seen from the comparison of Examples 1 to 6 with Comparative example 5 and the comparison of Examples 33 to 38 with Comparative example 6 that compared to an electrolyte including the cyclic sulfate compound represented by Formula III, an electrolyte including a cyclic sulfate compound represented by Formula I-1, I-2, I-6, I-7, I-8, or I-11 is conducive to increasing the number of cycles to capacity retention rate of 80% of the battery and ratios of the discharge capacities at the rates of 1.5C and 2C to the discharge capacity at 0.2C.

It can be seen from Examples 4, 7 to 10, 33, and 36 to 39 that controlling the mass content W of the cyclic sulfate compound based on the total mass of the electrolyte to satisfy 0.001% ≤ W ≤ 20% enables the battery to have a large number of cycles to capacity retention rate of 80% and high ratios of the discharge capacities at the rates of 0.2C, 1C, 1.5C, and 2C to the discharge capacity at 0.2C. It can be seen from the comparison of Examples 4, 8, and 9 with Examples 7 and 10 and the comparison of Examples 33, 37, and 38 with Examples 36 and 39 that further controlling the mass content W of the cyclic sulfate compound based on the total mass of the electrolyte to satisfy 0.1% ≤ W ≤ 5% is conducive to significantly increasing the ratio of the discharge capacity at the rate of 1C to the discharge capacity at 0.2C.

It can be seen from Examples 4 and 11 to 15 that controlling the mass content of the alkali metal ions based on the total mass of the electrolyte to be 30 ppm to 3000 ppm enables the battery to have a large number of cycles to capacity retention rate of 80% and high ratios of the discharge capacities at the rates of 0.2C, 1C, 1.5C, and 2C to the discharge capacity at 0.2C. It can be seen from the comparison of Examples 4 and 12 to 14 with Examples 11 and 15 that further controlling the mass content of the alkali metal ions to be 50 ppm to 2000 ppm is conducive to further increasing the ratio of the discharge capacity ratio at the rate of 1C to the discharge capacity at 0.2C.

It can be seen from Examples 4 and 16 to 20 that controlling the mass content of the high-valence metal ions based on the total mass of the electrolyte to be 15 ppm to 300 ppm enables the battery to have a large number of cycles to capacity retention rate of 80% and high ratios of the discharge capacities at the rates of 0.2C, 1C, 1.5C, and 2C to the discharge capacity at 0.2C.

It can be seen from Examples 4 and 21 to 26 that controlling the mass content of the alkaline earth metal ions based on the total mass of the electrolyte to be 13 ppm to 500 ppm enables the battery to have a large number of cycles to capacity retention rate of 80% and high ratios of the discharge capacities at the rates of 0.2C, 1C, 1.5C, and 2C to the discharge capacity at 0.2C.

It can be seen from the comparison of Examples 4 and 27 to 29 with Comparative example 1 and the comparison of Examples 40 and 41 with Comparative example 2 that the metal ion additives including sodium ions, copper ions, calcium ions, lithium ions, aluminum ions, or magnesium ions are all conducive to increasing the number of cycles to capacity retention rate of 80% of the battery and high ratios of the discharge capacities at the rates of 1C, 1.5C, and 2C to the discharge capacity at 0.2C.

It can be seen from Examples 4 and 42 to 44 that controlling the active specific surface area A of the negative electrode plate to be less than or equal to 20 cm²/g enables the battery to have a large number of cycles to capacity retention rate of 80% and high ratios of the discharge capacities at the rates of 0.2C, 1C, 1.5C, and 2C to the discharge capacity at 0.2C. It can be seen from the comparison of Examples 4, 42, and 43 with Example 44 that further controlling the active specific surface area of the negative electrode plate to be 5 cm²/g to 15 cm²/g is conducive to further increasing the number of cycles to capacity retention rate of 80% of the battery.

It should be noted that this application is not limited to the foregoing embodiments. The foregoing embodiments are merely examples, and embodiments having substantially the same constructions and the same effects as the technical idea within the scope of the technical solutions of this application are all included in the technical scope of this application. In addition, without departing from the essence of this application, various modifications made to the embodiments that can be conceived by persons skilled in the art, and other manners constructed by combining some of the constituent elements in the embodiments are also included in the scope of this application.

## Claims

1. An electrolyte, **characterized in that** the electrolyte comprises a cyclic sulfate compound represented by Formula I and a metal ion additive; 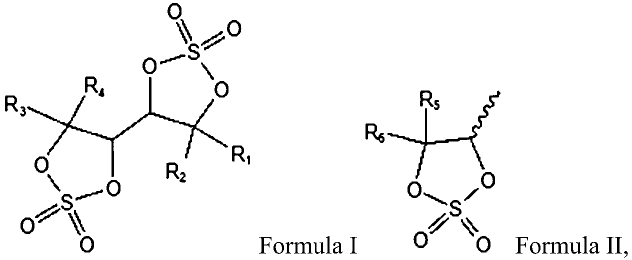
wherein in Formula I, R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and R₁, R₂, R₃, and R₄ are not all selected from a hydrogen atom; and
in Formula II, R₅ and R₆ are each independently selected from a hydrogen atom, a C₁-C₆ alkyl group, a halogen atom, a C₁-C₃ haloalkyl group, a C₁-C₃ alkoxy group, a C₁-C₃ haloalkoxy group, a double bond, an ester group, a cyano group, and a sulfonic acid group, and a wavy line indicates a bonding position.

2. The electrolyte according to claim 1, **characterized in that** R₁, R₂, R₃, and R₄ are each independently selected from a structure represented by Formula II, a hydrogen atom, a C₁-C₆ alkyl group, a halogen atom, a C₁-C₃ haloalkyl group, a C₁-C₃ alkoxy group, and a cyano group.

3. The electrolyte according to claim 1 or 2, **characterized in that**
R₁ is selected from a hydrogen atom and a C₁-C₃ alkyl group;
R₂ is selected from a C₁-C₃ alkyl group, a halogen atom, a C₁-C₃ haloalkyl group, a cyano group, or a structure represented by Formula II;
R₃ is selected from a hydrogen atom and a C₁-C₃ alkyl group; and
R₄ is selected from a C₁-C₃ alkyl group, a halogen atom, a C₁-C₃ alkoxy group, or a structure represented by Formula II.

4. The electrolyte according to any one of claims 1 to 3, **characterized in that**
R₁ is selected from a hydrogen atom or a methyl group;
R₂ is selected from a methyl group, an ethyl group, a fluorine atom, a trifluoromethyl group, a cyano group, or a structure represented by Formula II;
R₃ is selected from a hydrogen atom; and
R₄ is selected from a methyl group, an ethyl group, a propyl group, a fluorine atom, an ethoxy group, or a structure represented by Formula II.

5. The electrolyte according to any one of claims 1 to 4, **characterized in that**
R₁ and R₃ are each a hydrogen atom;
R₂ and R₄ are each a methyl group, an ethyl group, a fluorine atom, or a structure represented by Formula II; or
R₂ is a methyl group or a structure represented by Formula II, and R₄ is a methyl group.

6. The electrolyte according to any one of claims 1 to 5, **characterized in that**
R₁ and R₃ are each a hydrogen atom; and
R₂ and R₄ are each a methyl group or a structure represented by Formula II.

7. The electrolyte according to any one of claims 1 to 6, **characterized in that** R₅ and R₆ are each independently selected from a hydrogen atom and a C₁-C₃ alkyl group.

8. The electrolyte according to any one of claims 1 to 7, **characterized in that** the cyclic sulfate compound is selected from one or more of the following compounds:

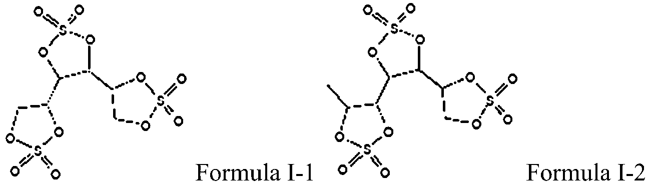

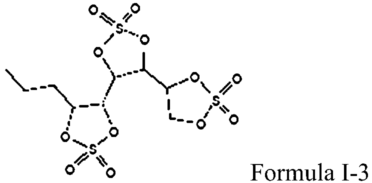

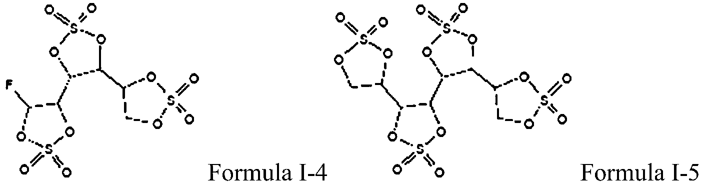

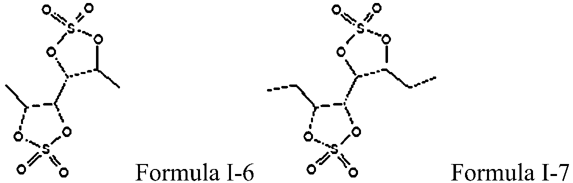

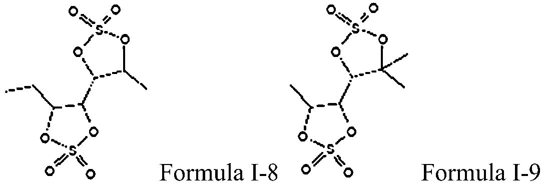

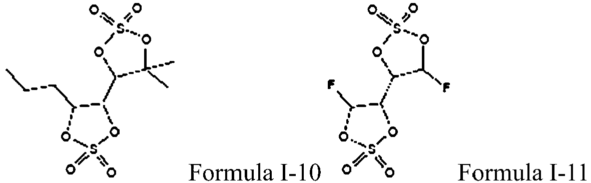

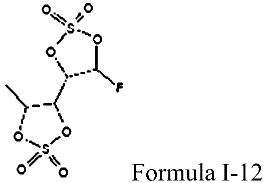

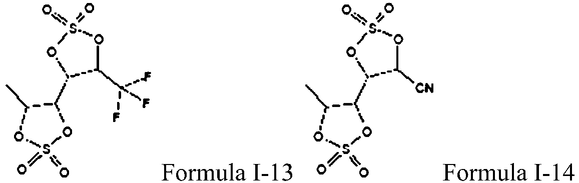

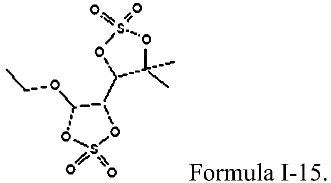

9. The electrolyte according to any one of claims 1 to 8, **characterized in that** based on a total mass of the electrolyte, a mass content W of the cyclic sulfate compound satisfies: 0.001% ≤ W ≤ 20%, optionally 0.1% to 5%.

10. The electrolyte according to any one of claims 1 to 9, **characterized in that** the metal ion additive comprises one or more of alkali metal ions, alkaline earth metal ions, and high-valence metal ions.

11. The electrolyte according to claim 10, **characterized in that** the alkali metal ions comprise one or more of sodium ions, lithium ions, and potassium ions; and/or
based on the total mass of the electrolyte, a mass content of the alkali metal ions is 30 ppm to 3000 ppm, optionally 50 ppm to 2000 ppm.

12. The electrolyte according to claim 10 or 11, **characterized in that** the high-valence metal ions comprise one or more of aluminum ions and copper ions; and/or
based on the total mass of the electrolyte, a mass content of the high-valence metal ions is 0.5 ppm to 300 ppm, optionally 15 ppm to 200 ppm.

13. The electrolyte according to any one of claims 10 to 12, **characterized in that** the alkaline earth metal ions comprise one or more of magnesium ions and calcium ions; and/or
based on the total mass of the electrolyte, a mass content of the alkaline earth metal ions is 0.5 ppm to 500 ppm, optionally 13 ppm to 400 ppm.

14. The electrolyte according to any one of claims 1 to 13, **characterized in that** the electrolyte further comprises an electrolytic salt, wherein the electrolytic salt comprises a lithium salt or a sodium salt;
the lithium salt comprises one or more of lithium hexafluorophosphate, lithium perchlorate, lithium tetrafluoroborate, lithium hexafluoroarsenate, lithium bis(fluorosulfonyl)imide, lithium bis(trifluoromethanesulfonyl)imide, lithium difluorobis(oxalato)phosphate, and lithium bis(oxalate)borate; and
the sodium salt comprises one or more of sodium hexafluorophosphate, sodium difluoro(oxalate)borate, sodium perchlorate, sodium bis(fluorosulfonyl)imide, sodium bis(trifluoromethanesulfonyl)imide, and sodium trifluoromethanesulfonate.

15. A secondary battery, **characterized by** comprising a negative electrode plate and the electrolyte according to any one of claims 1 to 14.

16. The secondary battery according to claim 15, **characterized in that** the secondary battery comprises a sodium battery or a lithium battery.

17. The secondary battery according to claim 15 or 16, **characterized in that** the negative electrode plate comprises a negative electrode active material, wherein the negative electrode active material comprises one or more of artificial graphite, natural graphite, hard carbon, soft carbon, mesocarbon microbeads, carbon fiber, carbon nanotubes, elemental silicon, a silicon-oxygen compound, a silicon-carbon composite, a silicon alloy, elemental tin, a tin-oxygen compound, and a titanium composite material.

18. The secondary battery according to any one of claims 15 to 17, **characterized in that** an active specific surface area A of the negative electrode plate and the mass content W of the cyclic sulfate compound satisfy: 0.0002 cm²/g ≤ W × A ≤ 0.04 cm²/g, optionally 0.02 cm²/g ≤ W × A ≤ 0.1 cm²/g.

19. The secondary battery according to claim 18, **characterized in that** the active specific surface area A of the negative electrode plate satisfies: A ≤ 20 cm²/g, optionally 5 cm²/g to 15 cm²/g.

20. An electric apparatus, **characterized by** comprising the secondary battery according to any one of claims 15 to 19.
